# EUROPEAN PATENT APPLICATION

(11) **EP 4 285 926 A1**
(43) Date of publication of application: **06.12.2023**
(21) Application number: 22176203.2
(22) Date of filing: 30.05.2022
(51) Int. Cl.: A61K 39/395, A61K 31/00, A61P 35/02, C07K 16/28, C07K 16/46

(54) **COMBINATION TREATMENT FOR CHRONIC LYMPHOCYTIC LEUKEMIA**

(71) Applicant: LAVA Therapeutics N.V., 3584 CM Utrecht (NL)
(72) Inventor: VAN DER VLIET, Johannes Jelle, 3584 CM Utrecht (NL); PARREN, Paul Willem Henri Ida, 3584 CM Utrecht (NL); RUBEN, Jurjen Matthijs, 3584 CM Utrecht (NL); LAMERIS, Roeland, 1081 HV Amsterdam (NL); DE GRUIJL, Tanja Denise, 1081 HV Amsterdam (NL); KATER, Arnon Philip, 1105 AZ Amsterdam (NL)
(74) Representative: Cooley (UK) LLP

(57) **Abstract**

The invention relates to the treatment of chronic lymphocytic leukemia, in particular to the treatment of chronic lymphocytic leukemia with a combination treatment comprising a bispecific antibody that binds CD1d and a Vγ9Vδ2-T cell receptor and an agonistic CD1d ligand.

## Description

### FIELD OF THE INVENTION

The present invention relates to the treatment of chronic lymphocytic leukemia (CLL), in particular to the treatment of CLL with a combination treatment comprising a bispecific antibody that binds CDld and a Vγ9Vδ2-T cell receptor and an agonistic CDld ligand.

### BACKGROUND OF THE INVENTION

CLL, previously also termed B-CLL, is the most common type of leukemia in the world. CLL affects B cell lymphocytes and causes malignant cells to proliferate and accumulate in the bone marrow and peripheral blood. Malignant CLL cells are distinguished from normal B lymphocytes by the expression of the CD5 surface marker. In recent years, the therapeutic arsenal for CLL has substantially increased with the introduction of tyrosine kinase inhibitors, Bcl-2 inhibitors and monoclonal antibodies. However, there is a need for novel treatment options, because of the need for long-term or continuous treatment and because of concerns relating to resistance and toxicity.

CDld (cluster of differentiation 1 d) has been identified as a potential target for antibody-based strategies in the treatment of hematological diseases, including CLL (de Weerdt et al. (2021) Clin Cancer Res 27:1744; WO2020060406).

CDld is a member of the CD1 family of glycoproteins and is expressed on the surface of various human cells, including antigen presenting cells (APC). CDld is a non-classical MHC protein involved in the presentation of lipid antigens to a subgroup of T cells. In humans, CDld is encoded by CD1D, also known as R3G1. APC displaying CDld include B-cells, dendritic cells (e.g. in lymph nodes), and monocytes. CDld is also expressed by various other cell types, for example, in liver, pancreas, skin, kidney, uterus, conjunctiva, epididymis, thymus and tonsil (Canchis et al. (1992) Immunology 80:561).

Cells that are activated/stimulated via CDld include CD1d-restricted Natural Killer T-cells (NKT cells). NKT cells are a heterogeneous group of T cells that share properties of both T cells and natural killer cells.

Type 1 or invariant NKT (iNKT) cells are the best-studied group of NKT cells and differ from conventional αβ-T cells in that their T-cell receptors are far more limited in diversity ('invariant'). These invariant, but also other CD1d-restricted, NKT cells (type 2 NKT) recognize several antigens, such as (self or foreign) lipids, glycolipids, sulfatides, phospholipids, lipopeptides, hydrophobic peptides and/or amphipathic α-helical peptides, presented by CDld molecules present on APC (Enrico Girardi et al. (2016) J Biol Chem. 291(20): 10677). Alpha-galactosylceramide (alpha-GalCer, also termed KRN7000), is the best studied ligand of the lipid-binding CDld in NKT cell activation in vitro and in vivo. The interaction between (antigen-presenting) CDld and TCR triggers the release of cytokines including Th1- and/or Th2-like cytokines, such as interferon gamma (IFN-γ), tumor necrosis factor-α, and interleukins, such as IL-4, IL-5 and IL- 13.

Important roles of type 1 NKT cells have been demonstrated in the regulation of autoimmune, allergic, antimicrobial, and antitumor immune responses (van der Vliet et al. (2004) Clin Immunol 112(1): 8). Physiologically, type 1 NKT cells can augment or inhibit immune responses, including antitumor, autoimmune, and anti-pathogen responses, through a variety of mechanisms depending on context (Yue et al. (2010) J Immunol 184: 268). In addition to cytokine release, NKT cell effector functions which result in cell lysis, such as perforin release and granzyme release and cell death, may also be relevant in conditions in which NKT cells are implicated, such as in cancer.

Selective activation of intrinsically tumor-responsive cytotoxic T lymphocytes represents an alternative approach that could generate a potent and focused anti-tumor response. Vγ9Vδ2-T cells form a conserved T cell subset that can induce apoptosis in a wide range of malignant cells in an HLA-independent way (Lo Presti et al. (2017) Front Immunol. 8:1401; de Weerdt et al. (2018) Blood 132(21):2260-2272; Gertner-Dardenne et al. (2012) J Immunol. 188(9):4701-4708; Kunzmann et al. (2000) Blood 96(2):384-392). This broad reactivity is understood to be conferred by phosphoantigens which are able to specifically activate this T-cell subset in a TCR dependent fashion. The broad antimicrobial and anti-tumor reactivity of Vγ9Vδ2-T cells suggest a direct involvement in immune control of cancers and infections. Hence, agents that can activate Vγ9Vδ2-T cells can be useful in the treatment of infections or cancer as these may promote Vγ9Vδ2-T cell reactivity towards the pathogen or infected cells or cancer.

WO2020060406 describes bispecific antibodies that bind CDld and the Vγ9Vδ2-T Cell Receptor (TCR). The antibodies were shown to engage Vγ9Vδ2-T cells, induce activation and degranulation of Vγ9Vδ2-T cells and thus lyse CD1d-expressing leukemic cells. Furthermore, a CDld binding VHH antibody (1D12) that can simultaneously contact CDld and a type 1 NKT TCR, thereby stabilizing this interaction through intrinsic bispecificity, has been described (Lameris et al. (2020 Nature Cancer 1:1054).

WO2020060405 describes a bispecific CD1d/Vγ9Vδ2-TCR antibody based on antibody 1D12 that can induce degranulation of Vγ9Vδ2-T cells as well as degranulation of type 1 NKT cells.

### SUMMARY OF THE INVENTION

It has now been found that in samples from CLL patients, the activation of type 1 NKT cells via a bispecific CD1d/Vγ9Vδ2-TCR antibody based on antibody 1D12 can be potentiated by addition of an exogenous agonistic CDld ligand.

In a first aspect, the invention relates to a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4 *for use in* the treatment of chronic lymphocytic leukemia in a human subject, wherein said treatment further comprises administration of an agonistic CDld ligand.

In a further aspect, the invention relates to a method for the treatment of chronic lymphocytic leukemia, comprising administration to a human subject in need thereof of:
(i) a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4, and
(ii) an agonistic CDld ligand.

### DETAILED DESCRIPTION OF THE FIGURES

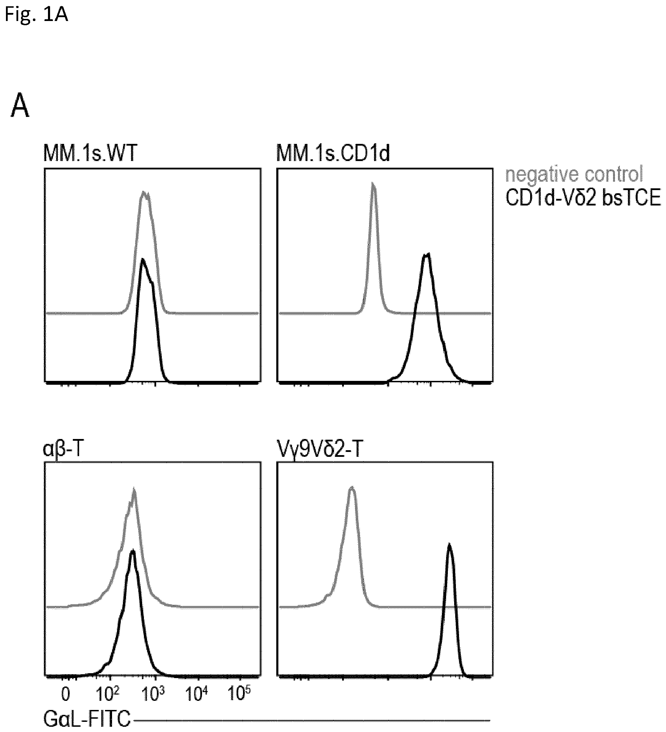

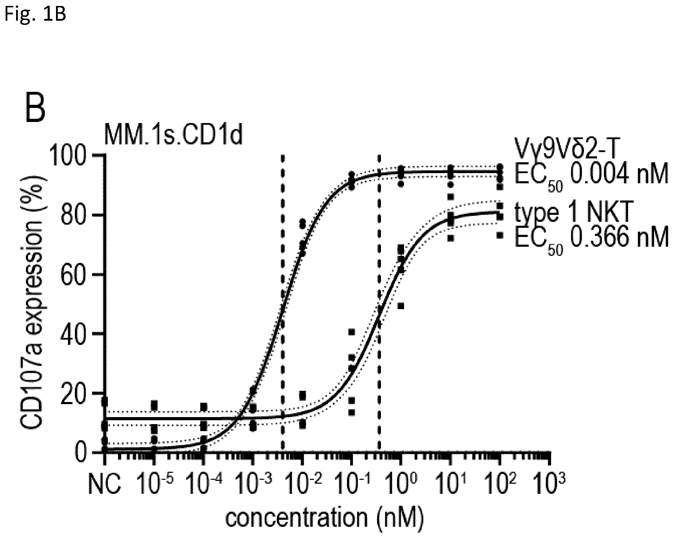

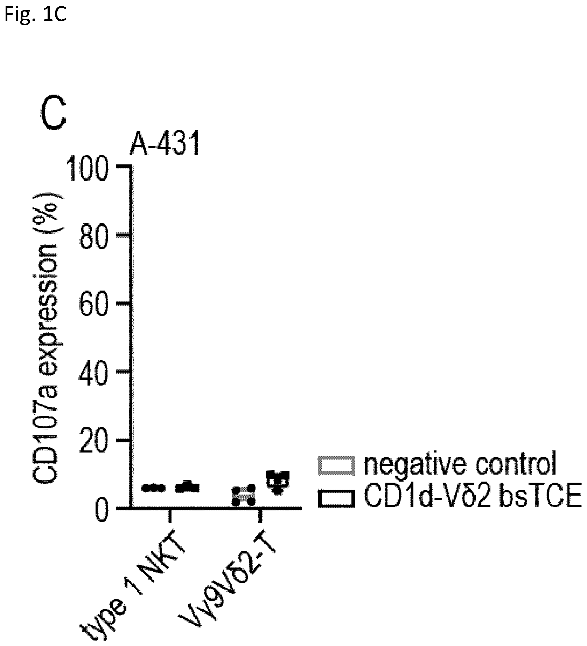

**Figure 1****. CD1d-Vδ2 bsTCE activates both type 1 NKT and Vγ9Vδ2-T cells. A,** Binding of CD1d-Vδ2 bsTCE to MM.1s.WT, MM.1s.CD1d, αβ-T cells or Vγ9Vδ2-T cells, detected by goat-anti-llama (GaL)-FITC. A representative histogram at 1000 nM is shown. **B,C,** Expression of CD107a on type 1 NKT and Vγ9Vδ2-T cells after 4 h co-culture with either MM.1s.CD1d cells **(B,** n=5) or CD1d-negative A-431 **(C,** type 1 NKT n=3, Vγ9Vδ2-T n=4) ± concentration range **(B)** or 100 nM **(C)** CD1d-Vδ2 bsTCE. NC, negative control. Box and whisker plots indicate the median, 25th to 75th percentiles and minimum to maximum. Non-linear regression with 95% confidence bands (dotted lines) and EC₅₀ₛ (dashed lines) **(B).**

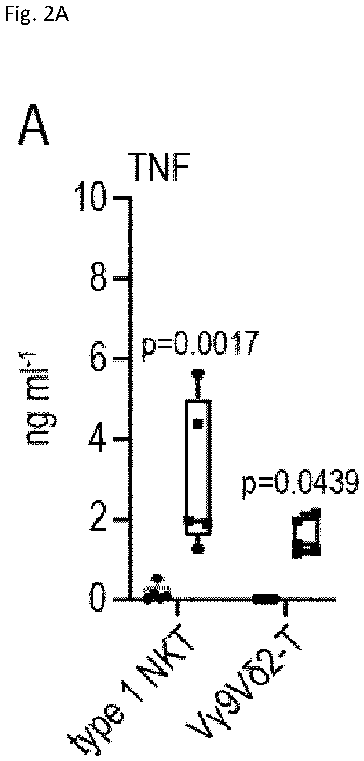

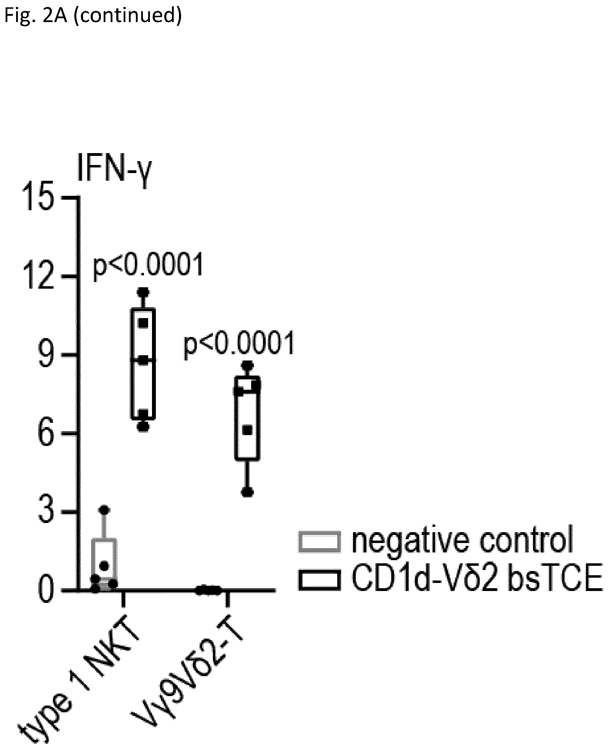

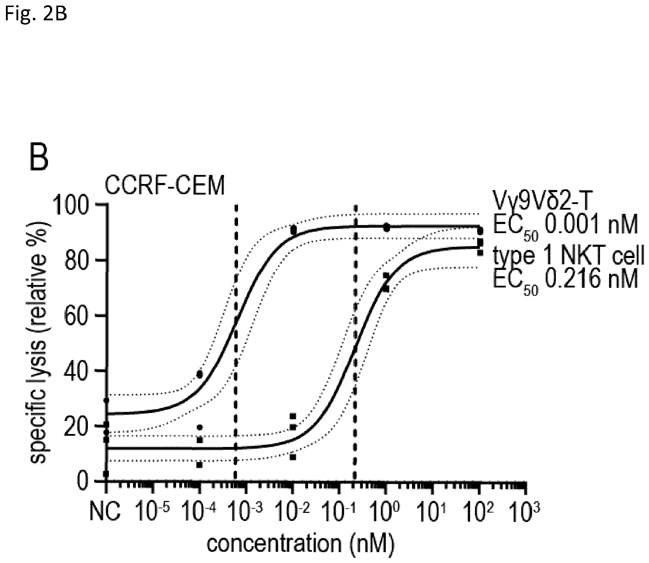

**Figure 2****. CD1d-Vδ2 bsTCE triggers both type 1 NKT and Vγ9Vδ2-T cell cytokine production, expansion and lysis of CD1d-expressing target cells. A,** Cytokine secretion by type 1 NKT or Vγ9Vδ2-T cells after 24 h co-culture with MM.1s.CD1d cells ± 50 nM CD1d-Vδ2 bsTCE (n=5). **B,** Specific lysis of CCRF-CEM (n=3) cells after 16 h co-culture with type 1 NKT or Vγ9Vδ2-T cells (E:T ratio 1:2) ± concentration range of CD1d-Vδ2 bsTCE. Box and whisker plots indicate the median, 25th to 75th percentiles and minimum to maximum. Two-way ANOVA with Šídák multiple comparisons test **(A).** Non-linear regression with 95% confidence bands (dotted lines) and EC₅₀ₛ (dashed lines) **(B).**

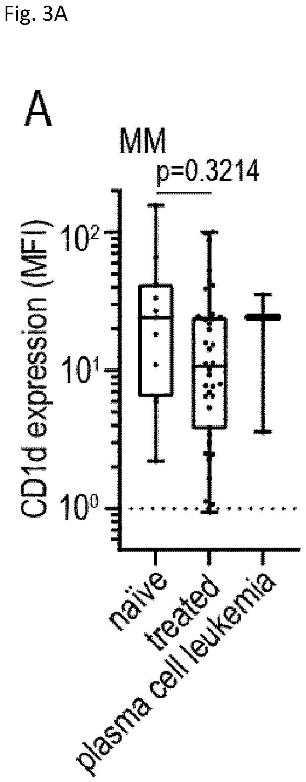

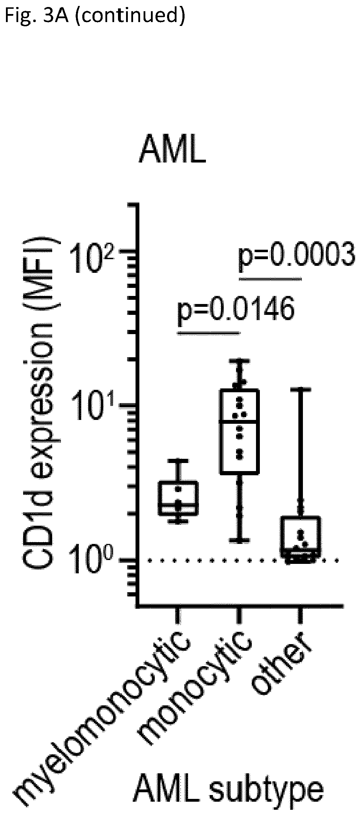

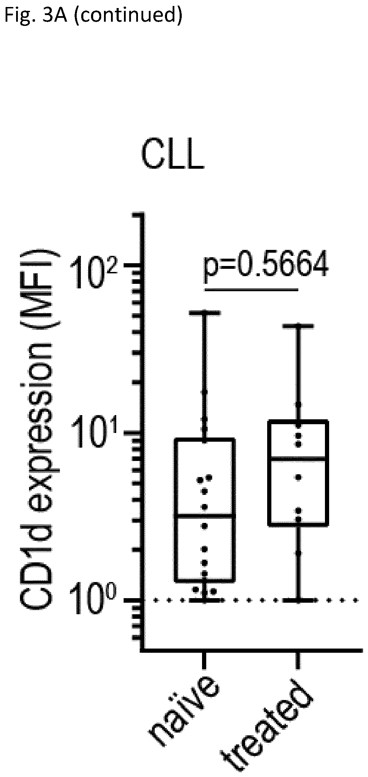

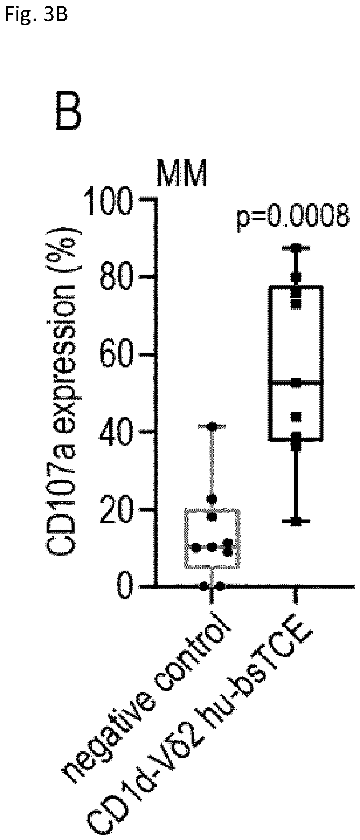

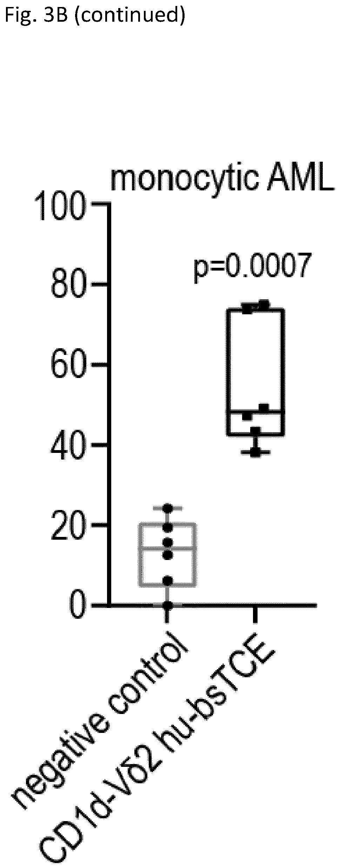

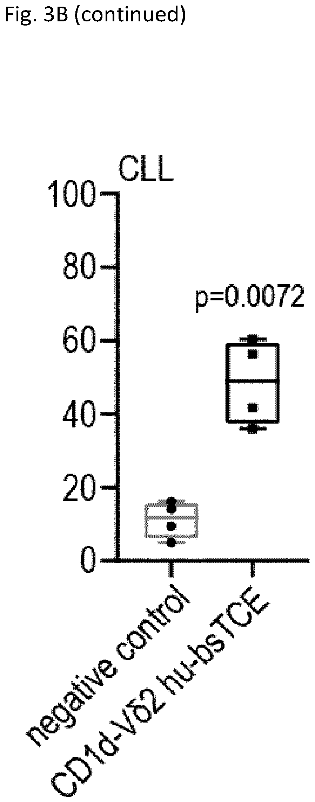

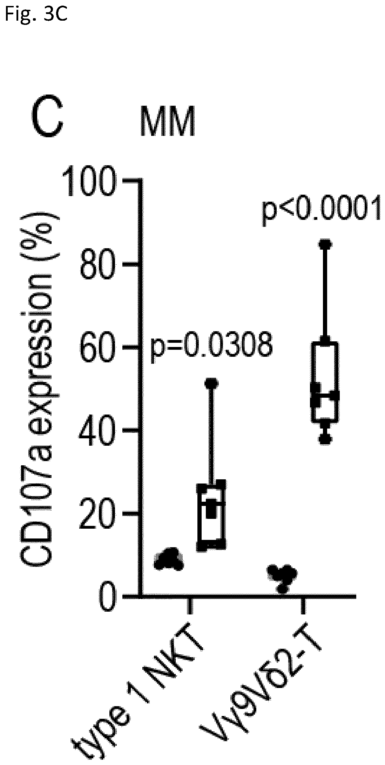

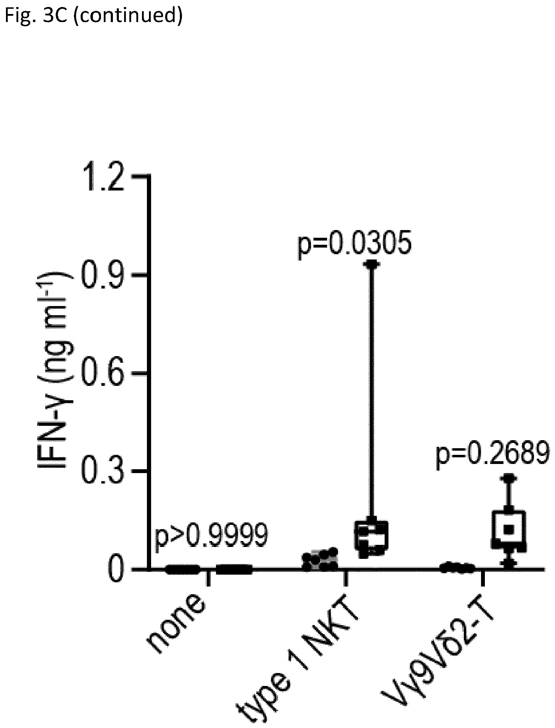

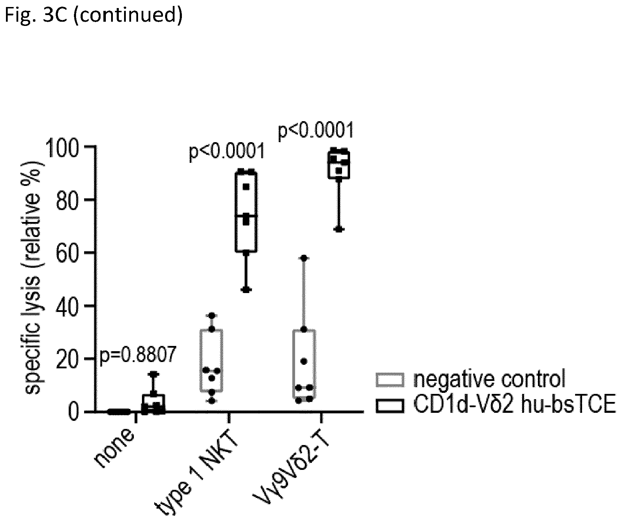

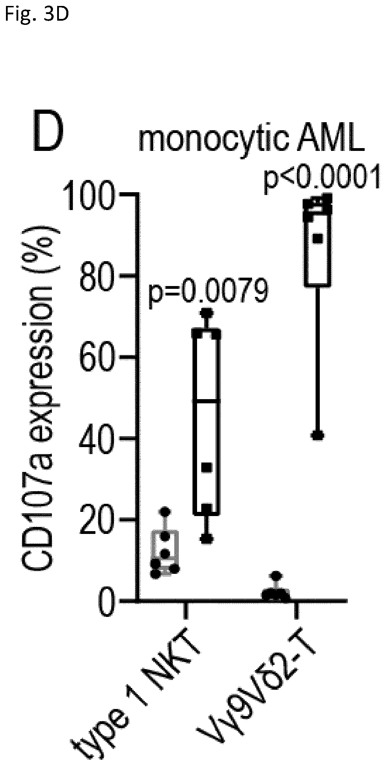

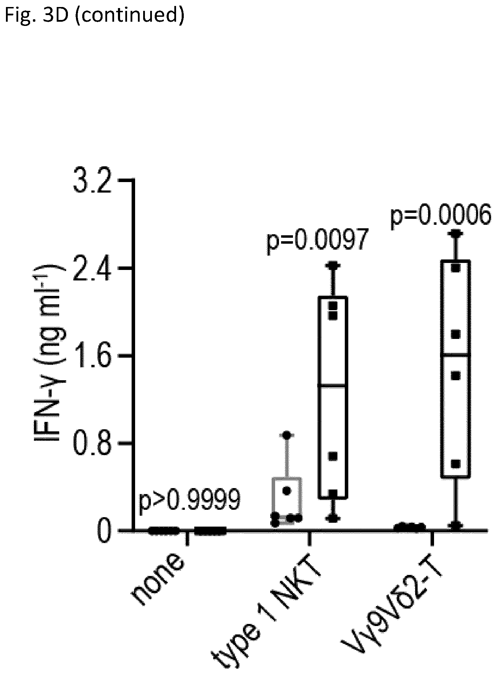

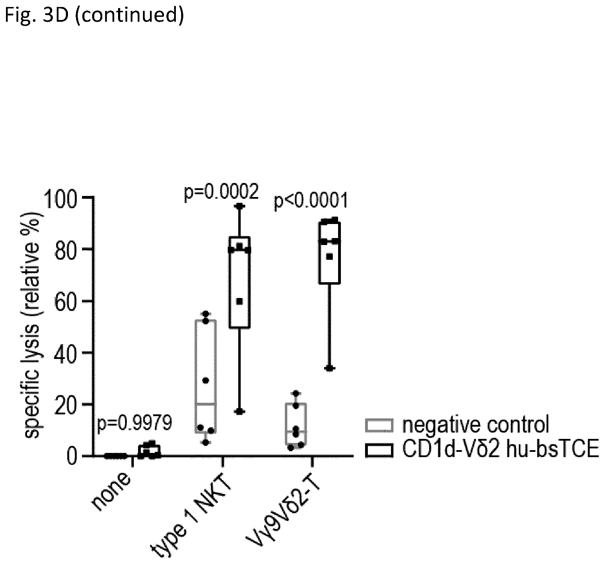

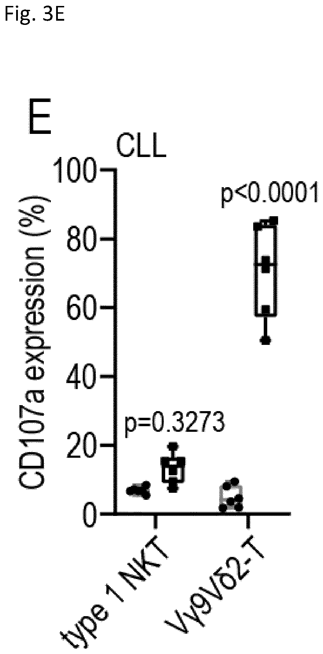

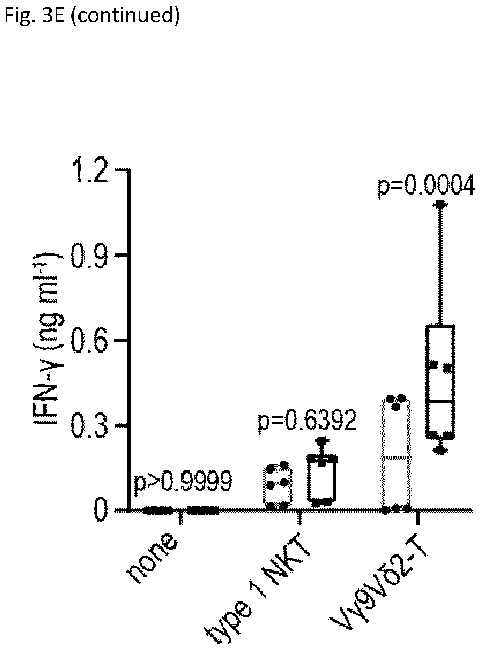

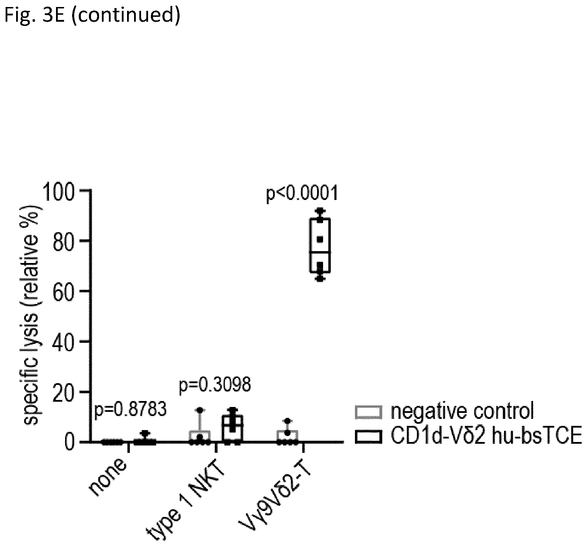

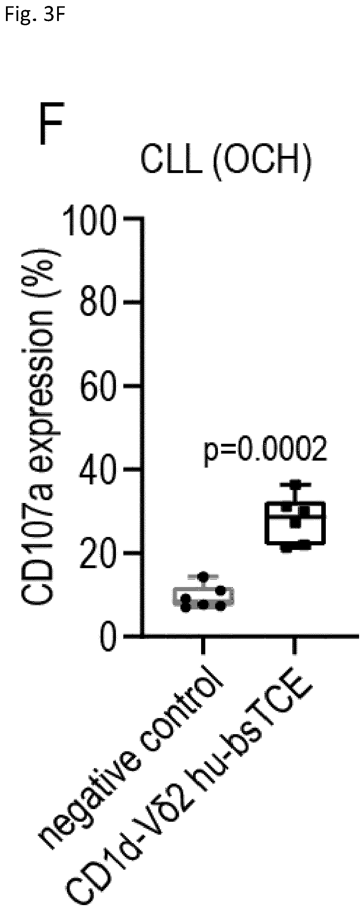

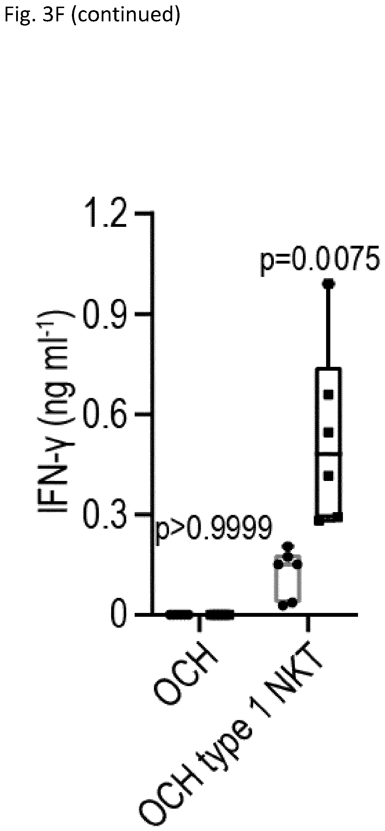

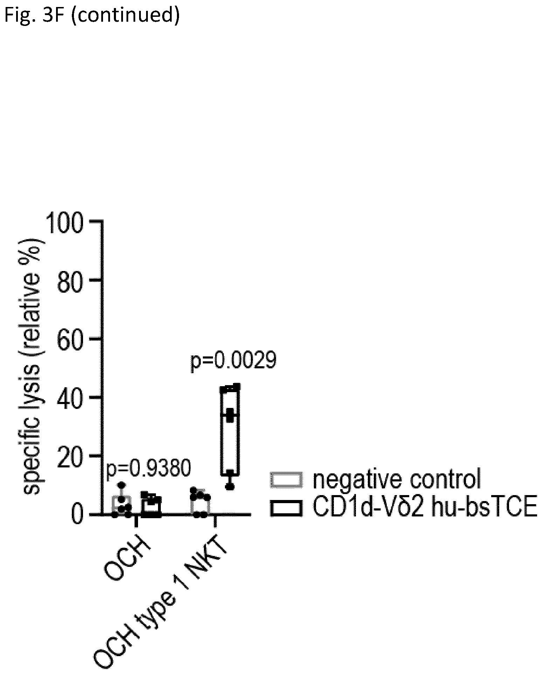

**Figure 3****. CD1d-Vδ2 bsTCE induces effector cell degranulation and cytotoxicity against patient-derived MM, monocytic AML and CLL cells. A,** CDld expression on patient MM (n=52), AML (n=38) and CLL cells (n=29) subdivided into treatment naïve and treated (MM and CLL) or subtype (AML). MFI, geometric mean fluorescence index. **B,** Expression of CD107a on autologous Vγ9Vδ2-T cells after 16 h culture of patient MM BMMC (n=9), monocytic AML BMMC (n=6) or CLL PBMC (n=4) ± 50 nM CD1d-Vδ2 hu-bsTCE. **C-F,** Expression of CD107a and IFN-γ secretion by allogeneic type 1 NKT or Vγ9Vδ2-T cells, and specific lysis of patient MM **(C,** n=7), monocytic AML **(D,** n=6) and CLL **(E,F,** n=6) cells after a 16 h (co-)culture of patient BMMC **(C,D)** or patient PBMC **(E,F)** (effector: BMMC/PBMC ratio 1:2) ± 50 nM CD1d-Vδ2 hu-bsTCE. **F,** patient CLL PBMC were pre-incubated with the synthetic glycolipid OCH (100 ng ml⁻¹) for 4 h. Box and whisker plots indicate the median, 25th to 75th percentiles and minimum to maximum. Unpaired two tailed T-test **(A),** paired two tailed T-test **(B,F),** one-way ANOVA with Tukey multiple comparisons test **(A),** two-way ANOVA with Šídák multiple comparisons test **(C-F).**

### DETAILED DESCRIPTION OF THE INVENTION

### Definitions

The term "human Vγ9Vδ2-T cell receptor" when used herein, refers to the Vγ9Vδ2-T cell receptor found on human γδ T cells.

The terms "δ2 chain" or "Vδ2", when used herein, refers to the rearranged δ2 chain of the Vγ9Vδ2-T cell receptor (TCR). GenBank: CAA51166.1, gives an example of a δ2 sequence. TRDV2, T cell receptor delta variable 2, represents the variable region (UniProtKB - A0JD36 (A0JD36_HUMAN) gives an example of a TRDV2 sequence). "binding the δ2 chain of a Vγ9Vδ2-TCR" means that the antibody can bind the δ2 chain as a separate molecule and/or as part of a Vγ9Vδ2-TCR (T cell receptor). However, the antibody will not bind to the γ9 chain as a separate molecule.

The terms "γ9 chain" or "Vγ9", when used herein, refers to the rearranged γ9 chain of the Vγ9Vδ2-T cell receptor (TCR). GenBank: NG_001336.2 gives an example of a γ9 sequence. TRGV9, T cell receptor gamma variable 9 represents the variable region (UniProtKB - Q99603_HUMAN gives an example of a TRGV9 sequence).

The term "CD1d", when used herein, refers to the human CDld protein (UniProtKB - P15813 (CD1D_HUMAN)) (SEQ ID NO:10).

The term "bsTCE" refers to a bispecific T Cell Engager, a bispecific antibody that has a binding specificity for a target on a γδ T cell and a further binding specificity for a target on a tumor cell. By binding to both these targets, γδ bsTCEs recruit γδ T cells to a tumor cell or tumor environment.

The term "immunoglobulin" as used herein is intended to refer to a class of structurally related glycoproteins typically consisting of two pairs of polypeptide chains, one pair of light (L) chains and one pair of heavy (H) chains, all four potentially inter-connected by disulfide bonds, although some mammalian species do not produce light chains and only make heavy-chain antibodies. The term "immunoglobulin heavy chain", "heavy chain of an immunoglobulin" or "heavy chain" as used herein is intended to refer to one of the chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The heavy chain constant region further comprises a hinge region. Within the structure of the immunoglobulin (e.g. IgG), the two heavy chains are inter-connected via disulfide bonds in the hinge region. Equally to the heavy chains, each light chain is typically comprised of several regions; a light chain variable region (VL) and a light chain constant region (CL). Furthermore, the VH and VL regions may be subdivided into regions of hypervariability (or hypervariable regions which may be hypervariable in sequence and/or form structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Choitia and Lesk (1987) J. Mol. Biol. 196:901 or Kabat et al. (1991) Sequence of protein of immunological interest, fifth edition. NIH publication. Various methods for CDR determination and amino acid numbering can be compared on www.abysis.org (UCL).

The term "antibody" is intended to refer to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about one hour, at least about two hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). Antigen-binding regions which interact with an antigen may comprise variable regions of both the heavy and light chains of an immunoglobulin molecule or may comprise or consist of single-domain antigen-binding regions, for example a heavy chain variable region only. The "Fc region" of an immunoglobulin is defined as the fragment of an antibody which would be typically generated after digestion of an antibody with papain which includes the two CH2-CH3 regions of an immunoglobulin and a connecting region, e.g. a hinge region. The constant domain of an antibody heavy chain defines the antibody isotype, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

The term "hinge region" as used herein is intended to refer to the hinge region of an immunoglobulin heavy chain. Thus, for example the hinge region of a human IgG1 antibody corresponds to amino acids 216-230 according to the EU numbering.

The term "CH2 region" or "CH2 domain" as used herein is intended to refer to the CH2 region of an immunoglobulin heavy chain. Thus, for example the CH2 region of a human IgG1 antibody corresponds to amino acids 231-340 according to the EU numbering. However, the CH2 region may also be any of the other subtypes as described herein.

The term "CH3 region" or "CH3 domain" as used herein is intended to refer to the CH3 region of an immunoglobulin heavy chain. Thus, for example the CH3 region of a human IgG1 antibody corresponds to amino acids 341-447 according to the EU numbering. However, the CH3 region may also be any of the other subtypes as described herein.

Reference to amino acid positions in the Fc region/Fc domain in the present invention is according to the EU-numbering (Edelman et al., Proc Natl Acad Sci U S A. 1969 May;63(1):78-85; Kabat et al., Sequences of proteins of immunological interest. 5th Edition - 1991 NIH Publication No. 91-3242).

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) that is encoded by heavy chain constant region genes. Each heavy chain isotype can be combined with either a kappa (κ) or lambda (λ) light chain. An antibody used in the invention can possess any isotype.

As indicated above, the term antibody as used herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include (i) a Fab' or Fab fragment, i.e. a monovalent fragment consisting of the VL, VH, CL and CH1 domains, or a monovalent antibody as described in WO2007059782; (ii) F(ab')2 fragments, i.e. bivalent fragments comprising two Fab fragments linked by a disulfide bridge at the hinge region; (iii) a Fd fragment consisting essentially of the VH and CH1 domains; and (iv) a Fv fragment consisting essentially of the VL and VH domains of a single arm of an antibody. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single-chain antibodies are encompassed within the term antibody unless otherwise indicated by context. The term antibody, unless specified otherwise, also includes polyclonal antibodies, monoclonal antibodies (mAbs), chimeric antibodies and humanized antibodies, and antibody fragments provided by any known technique, such as enzymatic cleavage, peptide synthesis, and recombinant techniques.

In some embodiments of the antibodies used in the invention, the first antigen-binding region or the second antigen-binding region, or both, is a single-domain antibody. Single-domain antibodies (sdAb, also called Nanobody^{®}, or VHH) are well known to the skilled person, see e.g. Hamers-Casterman et al. (1993) Nature 363:446, Roovers et al. (2007) Curr Opin Mol Ther 9:327 and Krah et al. (2016) Immunopharmacol Immunotoxicol 38:21. Single-domain antibodies comprise a single CDR1, a single CDR2 and a single CDR3. Examples of single-domain antibodies are variable fragments of heavy-chain-only antibodies, antibodies that naturally do not comprise light chains, single-domain antibodies derived from conventional antibodies, and engineered antibodies. Naturally occurring VHH molecules can be derived from antibodies raised in Camelidae species, for example in camel, dromedary, llama, alpaca and guanaco. Like a whole antibody, a single-domain antibody is able to bind selectively to a specific antigen. Single-domain antibodies may contain only the variable domain of an immunoglobulin chain, i.e. CDR1, CDR2 and CDR3 and framework regions.

The term "parent antibody", is to be understood as an antibody which is identical to an antibody used in the invention, but wherein the parent antibody does not have one or more of the specified mutations. A "variant" or "antibody variant" or a "variant of a parent antibody" used in the present invention is an antibody molecule which comprises one or more mutations as compared to a "parent antibody". Amino acid substitutions may exchange a native amino acid for another naturally-occurring amino acid, or for a non-naturally-occurring amino acid derivative. The amino acid substitution may be conservative or non-conservative. In the context of the present invention, conservative substitutions may be defined by substitutions within the classes of amino acids reflected in one or more of the following three tables:

### Amino acid residue classes for conservative substitutions

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

### Alternative conservative amino acid residue substitution classes

| | | | |
|---|---|---|---|
| 1 | A | S | T |
| 2 | D | E | |
| 3 | N | Q | |
| 4 | R | K | |
| 5 | I | L | M |
| 6 | F | Y | W |

### Alternative Physical and Functional Classifications of Amino Acid Residues

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, N, D, E, and R |

In the context of the present invention, a substitution in a variant is indicated as:
Original amino acid - position - substituted amino acid;
The three-letter code, or one letter code, are used, including the codes Xaa and X to indicate amino acid residue. Accordingly, the notation "T366W" means that the variant comprises a substitution of threonine with tryptophan in the variant amino acid position corresponding to the amino acid in position 366 in the parent antibody.

Furthermore, the term "a substitution" embraces a substitution into any one of the other nineteen natural amino acids, or into other amino acids, such as non-natural amino acids. For example, a substitution of amino acid T in position 366 includes each of the following substitutions: 366A, 366C, 366D, 366G, 366H, 366F, 366I, 366K, 366L, 366M, 366N, 366P, 366Q, 366R, 366S, 366E, 366V, 366W, and 366Y.

"% sequence identity", when used herein, refers to the number of identical nucleotide or amino acid positions shared by different sequences (i.e., % identity = # of identical positions/total # of positions × 100), taking into account the number of gaps, and the length of each gap, which need to be introduced for optimal alignment. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4.

In the context of the present invention, "competition" or "able to compete" or "compete" refers to any detectably significant reduction in the propensity for a particular antibody (e.g. a CDld antibody) to bind a particular binding partner (e.g. CD1d) in the presence of another molecule (e.g. a different CDld antibody) that binds the binding partner. Typically, competition means an at least about 25 percent reduction, such as an at least about 50 percent, e.g. an at least about 75 percent, such as an at least 90 percent reduction in binding between a CDld antibody or moiety, caused by the presence of another CDld antibody or moiety as determined by, e.g., ELISA analysis or flow cytometry using sufficient amounts of the two or more competing antibodies or moieties. Additional methods for determining binding specificity by competitive inhibition may be found in for instance Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc, and Wiley InterScience N. Y., (1992, 1993), and Muller, Meth. Enzymol. 92, 589-601 (1983)).

With "able to activate Vγ9Vδ2-T cells" in the context of the present invention is meant that Vγ9Vδ2-T cells are activated in the presence of the bispecific antibody, in particular in the presence of a target cell expressing CD1d. Preferably the activation of the Vγ9Vδ2-T cells is measurable through gene-expression and/or (surface) marker expression (e.g., activation markers, such as CD25, CD69, or CD107a) and/or secretory protein (e.g., cytokines or chemokines) profiles. In a preferred embodiment, the antibody is able to induce activation (e.g. upregulation of CD69 and/or CD25 expression) resulting in degranulation (marked by an increase in CD107a expression; see e.g. Example 3 of WO2020060405) and cytokine production (e.g. TNFα, IFN-γ) by Vγ9Vδ2-T cells. Preferably, the antibody that is used is able to increase CD107a expression on Vγ9Vδ2-T cells to at least 10%, more preferably at least 20%, more preferably at least 40%, most preferably at least 90%, when used in an assay as described in Example 3 of WO2020060405, wherein e.g. 10% means that 10% of the total number of cells is positive for CD107a. In another embodiment, the number of cells positive for CD107a is increased 1.5-fold, such as 2-fold, e.g. 5-fold, in the presence of an antibody used in the invention.

Similarly, for type 1 NKT cells, "able to activate" in the context of the present invention means that type 1 NKT cells behave differently in the presence of the bispecific antibody, in particular in the presence of a CDld molecule on a cell surface. Markers, such as CD25 (e.g. Example 1 of WO2020060405), CD69, CD107a (e.g. Example 3 of WO2020060405), or cytokines / chemokines, such as IFN-γ (e.g. Example 1 of WO2020060405), TNFα, IL-2, are used to determine whether type 1 NKT cells are activated. Preferably the bispecific antibody is able to increase CD107a expression on type 1 NKT cells to at least 10%, preferably at least 20%, more preferably to least 30%, most preferably to least 40%, when used in an assay as described in Example 3 of WO2020060405, wherein e.g. 10% means that 10% of the total number of cells is positive for CD107a. In another embodiment, the number of cells positive for CD107a is increased 1.5-fold, such as 2-fold, e.g. 5-fold, in the presence of an antibody used in the invention. Furthermore, preferably the bispecific antibody is able to increase CD25 expression on type 1 NKT cells to at least 10-fold, more preferably to at least 20-fold, most preferably to at least 30-fold compared to a "vehicle" control, as measured mean fluorescence intensity by flow cytometry, using conjugated CD25, e.g. allophycocyanin (APC)-conjugated CD25 in a FACS, when used in an assay as described in Example 1 of WO2020060405. Preferably, the bispecific antibody is able to increase IFN-γ expression by type 1 NKT cells at least 1.5-fold, such as at least 2-fold or at least 3-fold, e.g. when tested in an assay as described in Example 1 of WO2020060405.

The term "full-length antibody" when used herein, refers to an antibody which contains all heavy and light chain constant and variable domains corresponding to those that are normally found in a wild-type antibody of that isotype.

The term "chimeric antibody" refers to an antibody wherein the variable region is derived from a non-human species (e.g. derived from rodents) and the constant region is derived from a different species, such as human. Chimeric antibodies may be generated by genetic engineering. Chimeric monoclonal antibodies for therapeutic applications are developed to reduce antibody immunogenicity.

The term "humanized antibody" refers to a genetically engineered non-human antibody, which contains human antibody constant domains and non-human variable domains modified to contain a high level of sequence homology to human variable domains. This can be achieved by grafting of the non-human antibody complementarity-determining regions (CDRs), which together form the antigen binding site, onto a homologous human acceptor framework region (FR). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and, optionally, fully human constant regions. Optionally, additional amino acid modifications, which are not necessarily back-mutations, may be introduced to obtain a humanized antibody with preferred characteristics, such as affinity and biochemical properties. Humanization of non-human therapeutic antibodies is performed to minimize its immunogenicity in man while such humanized antibodies at the same time maintain the specificity and binding affinity of the antibody of non-human origin.

The term "multispecific antibody" refers to an antibody having specificities for at least two different, such as at least three, typically non-overlapping, epitopes, due to the presence of two or more antigen-binding regions. Such epitopes may be on the same or on different target antigens. If the epitopes are on different targets, such targets may be on the same cell or different cells or cell types.

The term "bispecific antibody" refers to an antibody having specificities for two different, typically non-overlapping, epitopes, due to the presence of two antigen-binding regions. Such epitopes may be on the same or different targets. If the epitopes are on different targets, such targets may be on the same cell or different cells or cell types.

Examples of different classes of bispecific antibodies include, but are not limited to, (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to an extra Fab fragment or parts of a Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab- fragments are fused together, fused to heavy-chain constant-domains, Fc-regions or parts thereof; and (vi) scFv-and diabody-based and heavy chain antibodies (e.g., domain antibodies, Nanobodies^{®}) wherein different single chain Fv molecules or different diabodies or different heavy-chain antibodies (e.g. domain antibodies, Nanobodies^{®}) are fused to each other or to another protein or carrier molecule fused to heavy-chain constant-domains, Fc-regions or parts thereof.

Examples of IgG-like molecules with complementary CH3 domains molecules include, but are not limited to, the Triomab^{®} (Trion Pharma/Fresenius Biotech), the Knobs-into-Holes (Genentech), CrossMAbs (Roche) and the electrostatically-matched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck,), mAb-Fv (Xencor), bivalent bispecific antibodies (Roche, WO2009080254) and DuoBody^{®} molecules (Genmab).

Examples of recombinant IgG-like dual targeting molecules include, but are not limited, to Dual Targeting (DT)-Ig (GSK/Domantis, WO2009058383), Two-in-one Antibody (Genentech, Bostrom, et al (2009). Science 323, 1610-1614), Crosslinked Mabs (Karmanos Cancer Center), mAb2 (F-Star), Zybodies^{™} (Zyngenia, LaFleur et al. MAbs. (2013) Mar-Apr;5(2):208-18), approaches with common light chain, κλBodies (NovImmune, WO2012023053) and CovX-body^{®} (CovX/Pfizer, Doppalapudi, V.R., et al (2007). Bioorg. Med. Chem. Lett. 17,501-506).

Examples of IgG fusion molecules include, but are not limited to, Dual Variable Domain (DVD)-Ig (Abbott), Dual domain double head antibodies (Unilever; Sanofi Aventis), IgG-like Bispecific (ImClone/Eli Lilly, Lewis et al. Nat Biotechnol. (2014) Feb;32(2): 191-8), Ts2Ab (MedImmune/AZ, Dimasi et al. J Mol Biol. (2009) Oct 30;393(3):672-92) and BsAb (Zymogenetics, WO2010111625), HERCULES (Biogen Idec), scFv fusion (Novartis), scFv fusion (Changzhou Adam Biotech Inc) and TvAb (Roche).

Examples of Fc fusion molecules include, but are not limited to, scFv/Fc Fusions (Academic Institution, Pearce et al Biochem Mol Biol Int. (1997) Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include, but are not limited to, F(ab)2 (Medarex/AMGEN), Dual-Action or Bis-Fab (Genentech), Dock-and-Lock^{®} (DNL) (ImmunoMedics), Bivalent Bispecific (Biotecnol) and Fab-Fv (UCB-Celltech).

Examples of scFv-, diabody-based and domain antibodies include, but are not limited to, Bispecific T Cell Engager (BiTE^{®}) (Micromet, Tandem Diabody (Tandab) (Affimed), Dual Affinity Retargeting Technology (DARTTM) (MacroGenics), Single-chain Diabody (Academic, Lawrence FEBS Lett. (1998) Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. (2010) Aug;88(6):667-75), dual targeting nanobodies^{®} (Ablynx, Hmila et al., FASEB J. (2010)), dual targeting heavy chain only domain antibodies.

In some embodiments, the multispecific antibody used in the invention is in a VHH-Fc format, i.e. the antibody comprises two or more single-domain antigen-binding regions that are linked to each other via a human Fc region dimer. In this format, each single-domain antigen-binding region is fused to an Fc region polypeptide and the two fusion polypeptides form a dimeric bispecific antibody via disulfide bridges in the hinge region. Such constructs typically do not contain full, or any, CH1 or light chain sequences. Figure 12B of WO06064136 provides an illustration of an example of this embodiment.

In the context of antibody binding to an antigen, the terms "binds", "capable of binding" or "specifically binds" refer to the binding of an antibody to a predetermined antigen or target (e.g. human Vδ2) to which binding typically is with an affinity corresponding to an apparent K_{D} of about 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less, e.g. when determined using flow cytometry as described in the Examples herein. Alternatively, K_{D} values can be determined using for instance surface plasmon resonance (SPR) technology in a BIAcore 3000 instrument using the antigen as the ligand and antibody as the analyte. Specific binding means that the antibody binds to the predetermined antigen with an affinity corresponding to a K_{D} that is at least ten-fold lower, such as at least 100-fold lower, for instance at least 1,000-fold lower, such as at least 10,000-fold lower, for instance at least 100,000-fold lower than its affinity for binding to a non-specific antigen (e.g., BSA, casein) other than the predetermined antigen or a closely-related antigen. The degree with which the affinity is lower is dependent on the K_{D} of the antibody, so that when the K_{D} of the antibody is very low (that is, the antibody is highly specific), then the degree with which the affinity for the antigen is lower than the affinity for a non-specific antigen may be at least 10,000-fold. The term "K_{D}" (M), as used herein, refers to the dissociation equilibrium constant of a particular interaction between the antigen and the antibody.

The terms "first" and "second" antigen-binding regions when used herein do not refer to their orientation / position in the antibody, i.e. they have no meaning with regard to the N- or C-terminus. The terms "first" and "second" only serve to correctly and consistently refer to the two different antigen-binding regions in the claims and the description.

The term "CD1d ligand" refers to a molecule that can be presented by, i.e. form a complex and be loaded on, CD1d. Many CDld ligands have been described in the art, see e.g. Brutkiewicz (2006) J Immunol 177:769 for a review. The term "agonistic CDld ligand" in the present context refers to a CDld ligand that, when presented in complex with CD1d, typically on a cell surface, can activate type 1 NKT cells.

The term "glycolipid" has its usual meaning in the art and refers to a lipid with a carbohydrate attached via a covalent bond.

### Multispecific antibodies

As described above, in a first aspect, the invention relates to a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4 and/or SEQ ID NO:9 for use in the treatment of chronic lymphocytic leukemia in a human subject, wherein said treatment further comprises administration of an agonistic CDld ligand.

In one embodiment, said multispecific antibody binds the same epitope on CDld as the antibody set forth in SEQ ID NO:4. The epitope binding of antibody 1D12 has been described in Lameris et al. (2020) Nature Cancer 1:1054 (incorporated by reference). In one embodiment, said multispecific antibody binds an epitope on CDld comprising one or more residues in the region from Phe76 to His86 and one or more residues in the region from Thr175 to Glu186 (SEQ ID NO:10). In a further embodiment, said multispecific antibody binds an epitope on CDld comprising one or more or all of the residues selected from the group consisting of Phe76, Ser77, Gln79, Gln80, Thr83, Leu84, His86, Thr175, Trp178, Thr183 and Gln186.

In a further embodiment, said multispecific antibody binds an epitope on CDld comprising one or more residues in the region from Phe76 to His86 and one or more residues in the region from Thr175 to Glu186, wherein said epitope comprises one or more, or all, of the residues selected from the group consisting of: Phe76, Ser77, Gln79, Gln80, Thr83, Leu84, His86, Thr175, Trp178, Thr183 and Gln186 and wherein said multispecific antibody binds an epitope on NKT12 TCRValpha (UniProtKB - A0A0B4J240 (TVA10_HUMAN)) which comprises one or more, or all, of the residues selected from the group consisting of: Phe73, Asn76, Thr86 and Asp90.

In one embodiment, said second antigen-binding region comprises or consists of a single domain antibody. In one embodiment, said second antigen-binding region comprises or consists of a humanized single domain antibody. In another embodiment, said second antigen-binding region comprises or consists of a chimeric single domain antibody.

In one embodiment, said multispecific antibody comprises the CDR1 sequence set forth in SEQ ID NO:1, the CDR2 sequence set forth in SEQ ID NO:2 and the CDR3 sequence set forth in SEQ ID NO:3.

In one embodiment, said second antigen-binding region comprises a single domain antibody comprising the CDR1 sequence set forth in SEQ ID NO:1, the CDR2 sequence set forth in SEQ ID NO:2 and the CDR3 sequence set forth in SEQ ID NO:3.

In one embodiment, said multispecific antibody comprises a sequence that is at least 80%, such as at least 85%, e.g. at least 90%, such as at least 95%, e.g. at least 96%, such as at least 97%, e.g. at least 98%, such as at least 99%, or 100% identical to the sequence set forth in SEQ ID NO:4.

In one embodiment, said multispecific antibody comprises a sequence that contains at most 10, such as at most 9, e.g. at most 8, such as at most 7, e.g. at most 6, such as at most 5, e.g. at most 4, such as at most 3, e.g. at most 2, such as at most 1 amino acid substitution as compared to the sequence set forth in SEQ ID NO:4. Preferably, the substitutions are conservative substitutions.

In one embodiment, said multispecific antibody comprises the sequence set forth in SEQ ID NO:4.

As described above, the multispecific antibody comprises a first antigen-binding region that binds a human Vγ9Vδ2-T cell receptor. In one embodiment, said multispecific antibody binds the δ2 chain of the human Vγ9Vδ2-T cell receptor. In another embodiment, said multispecific antibody binds the γ9 chain of the human Vγ9Vδ2-T cell receptor.

In one embodiment, said first antigen-binding region comprises or consists of a single domain antibody. In one embodiment, said first antigen-binding region comprises or consists of a humanized single domain antibody. In another embodiment, said first antigen-binding region comprises or consists of a chimeric single domain antibody.

In one embodiment, said multispecific antibody comprises the CDR1 sequence set forth in SEQ ID NO:5, the CDR2 sequence set forth in SEQ ID NO:6 and the CDR3 sequence set forth in SEQ ID NO:7.

In one embodiment, said first antigen-binding region comprises a single domain antibody comprising the CDR1 sequence set forth in SEQ ID NO:5, the CDR2 sequence set forth in SEQ ID NO:6 and the CDR3 sequence set forth in SEQ ID NO:7.

In one embodiment, X₁ is G. In another embodiment, X₁ is S. In one embodiment, X₂ is Y. In another embodiment, X₂ is F. In another embodiment, X₂ is S.

In one embodiment, said multispecific antibody comprises a sequence that is at least 80%, such as at least 85%, e.g. at least 90%, such as at least 95%, e.g. at least 96%, such as at least 97%, e.g. at least 98%, such as at least 99%, e.g. 100% identical to the sequence set forth in SEQ ID NO:8.

In one embodiment, said multispecific antibody comprises a sequence that contains at most 10, such as at most 9, e.g. at most 8, such as at most 7, e.g. at most 6, such as at most 5, e.g. at most 4, such as at most 3, e.g. at most 2, such as at most 1 amino acid substitution as compared to the sequence set forth in SEQ ID NO:8. Preferably, the substitutions are conservative substitutions.

In one embodiment, said multispecific antibody comprises or consists of the sequence set forth in SEQ ID NO:9.

In one embodiment, said is a bispecific antibody. In one embodiment, the antibody is a bispecific antibody and the two antigen-binding regions are directed or indirectly connected via a peptide linker, such as a linker of 4 glycines followed by a serine. In another embodiment, the antibody is a bispecific antibody in a VHH-Fc format, wherein, optionally, substitutions have been introduced to abolish Fc mediated effector function. For example, in one embodiment, the Fc region may comprise the substitutions L234F and/or L235E.

In one embodiment, said multispecific antibody is able to activate Vγ9Vδ2-T cells in the presence of a target cell expressing CD1d.

In one embodiment, said multispecific antibody is able to activate type 1 NKT cells in the presence of a target cell expressing CDld and more potently activate type 1 NKT cells in the presence of a target cell expressing CD1d, wherein an agonistic CDld ligand is complexed with CD1d.

In one embodiment, said multispecific antibody is able to activate type 1 NKT cells in the presence of a target cell expressing CDld complexed with the agonistic CDld ligand and said activation of type 1 NKT cells is increased as compared to the activation of type 1 NKT cells by the agonistic CDld ligand alone, i.e. without said multispecific antibody.

### Sequence listing (CDRs determined according to Kabat et al, supra):

| Name | Amino acid sequence | SEQ ID NO: |
|---|---|---|
| 1D12 CDR1 | DNVMG | 1 |
| 1D12 CDR2 | TIRTGGSTNYADSVKG | 2 |
| 1D12 CDR3 | TIPVPSTPYDY | 3 |
| 1D12 | QVQLVESGGGLVQAGGSLRLSCAASGSMFSDNVMGW YRQAPGKQRELVATIRTGGSTNYADSVKGRFTISRDNA KNTVYLQMNSLKPEDTAVYYCRHTIPVPSTPYDYWGQG TQVTVSS | 4 |
| 5C8 CDR1 | NYAMX₁, wherein X₁ is G or S | 5 |
| 5C8 CDR2 | AISWSGGSTSYADSVKG | 6 |
| 5C8 CDR3 | QFSGADX₂GFGRLGIRGYEYDY, wherein X₂ is Y, F or S | 7 |
| 5C8var | EVQLVESGGGLVQAGGSLRLSCAASGRPFSNYAM X₁WFRQAPGKEREFVAAISWSGGSTSYADSVKGRFTIS RDNAKNTVYLQMNSPKPEDTAIYYCAAQFSGAD X₂GFGRLGIRGYEYDYWGQGTQVTVSS, wherein X₁ is G or S and wherein X₂ is Y, F or S | 8 |
| 1D12-5C8var | EVQLVESGGGLVQAGGSLRLSCAASGSMFSDNVMGW YRQAPGKQRELVATIRTGGSTNYADSVKGRFTISRDNA KNTVYLQMNSLKPEDTAVYYCRHTIPVPSTPYDYWGQG TQVTVSSGGGGSEVQLLESGGGSVQPGGSLRLSCAAS GRPFSNYAMSWFRQAPGKEREFVSAISWSGGSTSYAD SVKGRFTISRDNSKNTLYLQMNSLRAEDTAVYYCAAQF SGADYGFGRLGIRGYEYDYWGQGTQVTVSS | 9 |
| Human CDld | MGCLLFLLLWALLQAWGSAEVPQRLFPLRCLQISSFANS SWTRTDGLAWLGELQTHSWSNDSDTVRSLKPWSQGT FSDQQWETLQHIFRVYRSSFTRDVKEFAKMLRLSYPLE LQVSAGCEVHPGNASNNFFHVAFQGKDILSFQGTSWE | 10 |
| | PTQEAPLWVNLAIQVLNQDKWTRETVQWLLNGTCPQF VSGLLESGKSELKKQVKPKAWLSRGPSPGPGRLLLVCH VSGFYPKPVWVKWMRGEQEQQGTQPGDILPNADETW YLRATLDVVAGEAAGLSCRVKHSSLEGQDIVLYWGGSY TSMGLIALAVLACLLFLLIVGFTSRFKRQTSYQGVL | |

### Agonistic CDld ligands

The agonistic CDld ligand used in the invention can form a complex with human CD1d. In one embodiment, a complex of human CDld and said agonistic CDld ligand presented on CD1d-expressing cells is capable of activating type 1 NKT cells.

In one embodiment, a complex of human CDld and said agonistic CDld ligand presented on CD1d-expressing cells is capable of inducing production of IFN-γ and/or IL-4 by type 1 NKT cells, e.g. as determined using the methods described in Miyamoto et al. (2010) Nature 413: 531 (e.g. Figure 1d), the methods described in Jukes et al (2016) Eur J Immunol 46:1224, the methods described in Jervis et al. (2012) Bioorg Med Chem Lett 22:4348 or the methods described in Li et al. (2008) Immunol 127:216 (all incorporated by reference).

In another embodiment, the complex of human CDld and said agonistic CDld ligand presented on CD1d-expressing cells is capable of inducing proliferation of type 1 NKT cells in vitro, for example as determined by proliferation of splenocytes in the presence of an agonistic CDld ligand, e.g. as determined using the method described in Miyamoto et al. (2010) Nature 413: 531 (Figure 1c).

In one embodiment, the agonistic CDld ligand is a glycolipid. In one embodiment, the agonistic CDld ligand is alpha-GalCer.

In another embodiment, the agonistic CDld ligand is an alpha-GalCer analogue, such as an alpha-GalCer analogue set forth in Jervis et al. (2012) Bioorg Med Chem Lett 22:4348 (incorporated by reference). In one embodiment the agonistic CDld ligand is a 6"-derivatised alpha-GalCer analogue, such as a 6"-triazole-substituted alpha-GalCer analogue. In one embodiment, the agonistic CDld ligand is selected from analogues 3, 4, 5, 6, 7, 8, 9, 10, 11, 12a, 12b, 12c, 12d, 12e, 12f, 13, 14 and 15, set forth in Jervis et al. (2012) Bioorg Med Chem Lett 22:4348.

In one embodiment, the agonistic CDld ligand is an alpha-GalCer analogue as set forth in Velmourougane (2009) Bioorg Med Chem Lett 19:3386 (incorporated by reference). In one embodiment, the agonistic CDld ligand is selected from analogues 1, 2, 3 and 4 set forth in Velmourougane (2009) Bioorg Med Chem Lett 19:3386 (Figure 2).

In one embodiment, the agonistic CDld ligand is an alpha-GalCer analogue as set forth in Bricard et al. (2010) PloS 5:e14374. In one embodiment the agonistic CDld ligand is selected from the group consisting of: C-glycoside, C20:2, C20:4, Lyso, Toluenoyl, Difluorobenzoyl, Bromobenzoyl, and Adamantanoyl, set forth in Bricard et al. (2010) PloS 5:e14374 (Figure 1).

In one embodiment, the agonistic CDld ligand is an alpha-GalCer analogue as set forth in Chennamadhavuni et al. (2018) Cell Chem Biol 25:571 (incorporated by reference). In one embodiment, the agonistic CDld ligand is selected from the group consisting of: alpha-C-GalCer, AH03-1, NU-alpha-GalCer, PyrC-alpha-GalCer, alpha-GalCer C20:2, AH10-7 and AH15-1, set forth in Chennamadhavuni et al. (2018) Cell Chem Biol 25:571 (Figure 1).

In one embodiment, the agonistic CDld ligand is F-2/S-3 or F-2/S-7, set forth in Oki et al (2004) J Clin Invest 113:1622 (Figure 2A) (incorporated by reference).

In another embodiment, the agonistic CDld ligand is 7DW8-5 or alpha-C-GalCer, reviewed by Carreño et al (2016) Clin Trans Immunol 5, e69 (incorporated by reference).

In another embodiment, the agonistic CDld ligand is beta-GalCer (beta-galactosylceramide) or an analogue thereof. In one embodiment, the agonistic CDld ligand is selected from the group consisting of: beta-GalCer, beta-LacCer, iGb3 and Gb3, set forth in Pellicci et al (2011) Nature Immunol 9:827 (incorporated by reference).

In another embodiment, the agonistic CDld ligand is OCH, described in Miyamoto et al. (2010) Nature 413: 531 (incorporated herein by reference).

In another embodiment, the agonistic CDld ligand is 3',4"-deoxy-alpha-GalCer, see e.g. Raju et al. (2009) Bioorg Med Chem Lett 19:4122; Lameris et al. (2020) Nature Cancer 1:1054.

In another embodiment, the agonistic CDld ligand is a sphingosine chain analogue of alpha-GalCer, such as OCH, C13, Trazole 8 or RCAI-18, described in Venkataswamya and Porcelli (2010) Sem Immunol 22:68 (incorporated by reference).

In another embodiment, the agonistic CDld ligand is an N-acyl substituted analogue of alpha-GalCer, such as C20:2, C20:4, PGB1, C10:0, 4FPA or C11Ph, described in Venkataswamya and Porcelli (2010) Sem Immunol 22:68.

In another embodiment, the agonistic CDld ligand is a glycosidic bond derivative analogue of alpha-GalCer, such as alpha-C-GalCer or GSK109, described in Venkataswamya and Porcelli (2010) Sem Immunol 22:68.

In another embodiment, the agonistic CDld ligand is a carbohydrate modified analogue of alpha-GalCer, such as Gal(alphal-6)alpha-GalCer or 6'-ureido-1-naphtyl, described in Venkataswamya and Porcelli (2010) Sem Immunol 22:68.

In one embodiment, the agonistic CDld ligand comprises the structure: 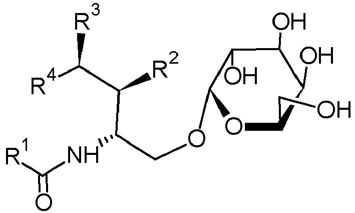
wherein R¹ is a C20-C30 alkyl group, preferably a linear C20-C30 alkyl group, e.g. a linear C25 group or a 11-(4-fluorophenyl)undecanoyl group,
wherein R² is H or OH,
wherein R³ is H or OH, and
wherein R⁴ is a C4-C18 alkyl group, preferably a linear C4-C18 alkyl group, e.g. a linear C5 alkyl group, a linear C14 alkyl group or a linear C15 alkyl group.

In another embodiment, the agonistic CDld ligand is not a glycolipid. Non-glycolipid CDld ligands have, for example, been described in Silk et al. (2008) J Immunol 180:6452 and Jukes et al (2016) Eur J Immunol 46:1224 (both hereby incorporated by reference).

In one embodiment, the agonistic CDld ligand is ThrCer (threitolceramide), AraCer (arabitinitolceramide) or GlyCer (glycerolceramide) (Silk et al. (2008) J Immunol 180:6452).

In another embodiment, the agonistic CDld ligand is ThrCer 6 or ThrCer 7 (described in Jukes et al (2016) Eur J Immunol 46:1224).

In one embodiment, the agonistic CDld ligand is selected from the group consisting of: alpha-GalCer, ThrCer, 7DW8-5 and OCH.

In one embodiment, the agonistic CDld ligand used in the invention is a weaker agonist for type 1 NKT cells than alpha-GalCer.

In one embodiment, the K_{D} for binding of a complex of human CDld loaded with said agonistic CDld ligand to a type 1 NKT T Cell Receptor is higher than the K_{D} for binding of a complex of human CDld loaded with alpha-GalCer to a type 1 NKT T Cell Receptor. This can e.g. be evaluated by determining binding to a soluble form of a type 1 NKT T Cell Receptor. Thus, in one embodiment, the K_{D} for binding of a complex of human CDld loaded with the agonistic CDld ligand to a soluble type 1 NKT T Cell Receptor is higher than the K_{D} for binding of a complex of human CDld loaded with alpha-GalCer to a soluble type 1 NKT T Cell Receptor. This may, for example, be tested as described in Silk et al. (2008) J Immunol 180:6452; Jukes et al (2016) Eur J Immunol 46:1224 or MacCarthy et al (2007) JEM 204:1131. In one embodiment, said K_{D}, is at least 2-fold higher, such as at least 3, at least 4, at least 5 or at least 10-fold higher than the corresponding K_{D} for alpha-GalCer.

In one embodiment, the agonistic CDld ligand used in the invention has a reduced ability to induce IFN-γ production by type 1 NKT cells as compared to alpha-GalCer. This may, for example, be tested by co-culturing of type 1 NKT cells with a CD1d-expressing HeLa cell line in the presence of the agonistic CDld ligand. A suitable method has e.g. been described in Li et al. (2008) Immunol 127:216 (incorporated by reference). In one embodiment, the ability to induce IFN-γ production by type 1 NKT cells is at least 2-fold lower, such as at least 3, at least 4, at least 5 or at least 10-fold lower than that of alpha-GalCer.

In one embodiment, the agonistic CDld ligand used in the invention has a reduced ability to induce IL-4 production by type 1 NKT cells as compared to alpha-GalCer. This may, for example, be tested by co-culturing of type 1 NKT cells with a CD1d-expressing HeLa cell line in the presence of the agonistic CDld ligand. A suitable method has e.g. been described in Li et al. (2008) Immunol 127:216 (incorporated by reference). In one embodiment, the ability to induce IL-4 production by type 1 NKT cells is at least 2-fold lower, such as at least 3, at least 4, at least 5 or at least 10-fold lower than that of alpha-GalCer.

In another embodiment, the agonistic CDld ligand used in the invention is a stronger agonist for type 1 NKT cells than alpha-GalCer.

In one embodiment, the K_{D} for binding of a complex of human CDld loaded with said agonistic CDld ligand to a type 1 NKT T Cell Receptor is lower than the K_{D} for binding of a complex of human CDld loaded with alpha-GalCer to a type 1 NKT T Cell Receptor. This can e.g. be evaluated by determining binding to a soluble form of a type 1 NKT T Cell Receptor. Thus, in one embodiment, the K_{D} for binding of a complex of human CDld loaded with the agonistic CDld ligand to a soluble type 1 NKT T Cell Receptor is lower than the K_{D} for binding of a complex of human CDld loaded with alpha-GalCer to a soluble type 1 NKT T Cell Receptor. This may, for example, be tested as described in Silk et al. (2008) J Immunol 180:6452; Jukes et al (2016) Eur J Immunol 46:1224 or MacCarthy et al (2007) JEM 204:1131. In one embodiment, said K_{D}, is at least 2-fold higher, such as at least 3, at least 4, at least 5 or at least 10-fold lower than the corresponding K_{D} for alpha-GalCer.

In one embodiment, the agonistic CDld ligand used in the invention has an increased ability to induce IFN-γ production by type 1 NKT cells as compared to alpha-GalCer. This may, for example, be tested by co-culturing of type 1 NKT cells with a CD1d-expressing HeLa cell line in the presence of the agonistic CDld ligand. A suitable method has e.g. been described in Li et al. (2008) Immunol 127:216 (incorporated by reference). In one embodiment, the ability to induce IFN-γ production by type 1 NKT cells is at least 2-fold higher, such as at least 3, at least 4, at least 5 or at least 10-fold higher than that of alpha-GalCer.

In one embodiment, the agonistic CDld ligand used in the invention has an increased ability to induce IL-4 production by type 1 NKT cells as compared to alpha-GalCer. This may, for example, be tested by co-culturing of type 1 NKT cells with a **CD1d-expressing** HeLa cell line in the presence of the agonistic CDld ligand. A suitable method has e.g. been described in Li et al. (2008) Immunol 127:216 (incorporated by reference). In one embodiment, the ability to induce IL-4 production by type 1 NKT cells is at least 2-fold higher, such as at least 3, at least 4, at least 5 or at least 10-fold higher than that of alpha-GalCer.

CDld ligands are known to vary in the type of response that they induce in type 1 NKT cells, see e.g. East et al. (2014) Med Res Rev 34:45. Some ligands, e.g. OCH, induce a more Th2 biased response, characterized by, e.g., a relatively high production of IL-4. Other ligands, e.g. alpha-C-GalCer, induce a more Th1 biased response, characterized by, e.g., a relatively high production of IFN-γ.

In some embodiments, a bias towards a Th1 response is desired. In one embodiment, the agonistic CDld ligand used in the invention induces a more Th1-biased response by type 1 NKT cells compared to OCH. In another embodiment, the agonistic CDld ligand used in the invention induces a more Th1-biased response by type 1 NKT cells compared to alpha-GalCer. Relative Th1/Th2 biases may be determined by comparing the IFN-γ/IL-4 induction ratio for a ligand with the IFN-γ/IL-4 induction ratio for the reference compound, such alpha-GalCer. Suitable methods for determining IFN-γ/IL-4 induction ratios have e.g. been described in Jervis et al. (2012) Bioorg Med Chem Lett 22:4348 and in Li et al. (2008) Immunol 127:216.

In other embodiments, a bias towards a Th2 response is desired. In one embodiment, the agonistic CDld ligand used in the invention induces a more Th2-biased response by type 1 NKT cells compared to alpha-C-GalCer. In another embodiment, the agonistic CDld ligand used in the invention induces a more Th1-biased response by type 1 NKT cells compared to alpha-GalCer. Relative Th1/Th2 biases may be determined by comparing the IFN-γ/IL-4 induction ratio for a ligand with the IFN-γ/IL-4 induction ratio for the reference compound, such alpha-GalCer. Suitable methods for determining IFN-γ/IL-4 induction ratios have e.g. been described in Jervis et al. (2012) Bioorg Med Chem Lett 22:4348 and in Li et al. (2008) Immunol 127:216.

In one embodiment, said agonistic CDld ligand is capable of becoming loaded on CDld when CDld is located on the cell surface, i.e. the agonistic CDld ligand can form a complex with a CDld molecule that is already present on the cell surface and the loading of the ligand on CDld does not necessarily have to take place intracellularly. In one embodiment, the agonistic CDld ligand is (also) capable of being loaded intracellularly on CD1d.

### Methods of treatment

As described above, in a first aspect, the invention relates to a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4 *for use in* the treatment of chronic lymphocytic leukemia in a human subject, wherein said treatment further comprises administration of an agonistic CDld ligand.

In a further aspect, the invention relates to a method for the treatment of chronic lymphocytic leukemia, comprising administration to a human subject in need thereof of:
(i) a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4, and
(ii) an agonistic CDld ligand.

Thus, the method of the invention relates to a combination therapy comprising administration of a multispecific antibody as defined herein and an agonistic CDld ligand.

In one embodiment, the treatment with said agonistic CDld ligand is initiated prior to, or simultaneously with, the treatment with the multispecific antibody.

In one embodiment, the multispecific antibody is administered intravenously. In another embodiment, the multispecific antibody is administered subcutaneously.

In one embodiment, the agonistic CDld ligand is administered parenterally, such as intravenously., intramuscularly or subcutaneously. In another embodiment, the agonistic CDld ligand is delivered using a vector, such as a nanoparticle, an exosome or a liposome.

In one embodiment, the treatment comprises administration of the multispecific antibody more than once, such as between 2 and 100 times, e.g. between 2 and 50 times, e.g. between 2 and 25 times, such as between 6 and 25 times, or between 6 and 12 times, for example twice weekly, weekly, biweekly or with an interval of 3 to 4 weeks.

In one embodiment, the treatment comprises administration of the agonistic CDld ligand more than once, such as between 2 and 100 times, e.g. between 2 and 50 times, e.g. between 2 and 25 times, such as between 6 and 25 times, or between 6 and 12 times, for example, daily, twice weekly, weekly, biweekly or with an interval of 3 to 10 weeks, such as 4 or 8 weeks.

### EXAMPLES

### Example 1: Materials and Methods

### Cell lines

The T-ALL cell line CCRF-CEM was obtained from the ATCC and grown in RPMI-1640 supplemented with 10% (v/v) fetal calf serum, 0.05 mM β-mercaptoethanol, 100 IU ml⁻¹ sodium penicillin, 100 µg ml⁻¹ streptomycin sulphate and 2.0 mM L-glutamine. The MM cell line MM.1s, wild type (WT) whether or not transduced with the lentiviral plasmid FUW-mCherry/luc and electroporated with full length single chain β2m-CD1d pcDNA3.1 vector (referred to as MM.1s.WT and MM.1s.CD1d) (Im et al. (2004) J Biol Chem 279:299) were grown in RPMI-1640 supplemented with 10% (v/v) fetal calf serum, 0.05 mM β-mercaptoethanol, 100 IU ml⁻¹ sodium penicillin, 100 µg ml⁻¹ streptomycin sulphate and 2.0 mM L-glutamine. The epithelial cell line A-431 was obtained from the ATCC and grown in DMEM supplemented with 10% (v/v) FCS, 0.05 mM of β-mercaptoethanol, 100 IU ml⁻¹ of sodium penicillin, 100 µg ml⁻¹ of streptomycin sulfate and 2.0 mM of L-glutamine. Cell lines were confirmed to be Mycoplasma-free. Authentication of CCRF-CEM and MM.1s transfectants (BaseClear and Eurofins) showed no signs of contamination or misidentification.

### Human samples

PBMC from healthy donors were isolated from venous blood by density gradient centrifugation, obtained from Sanquin blood supply under written informed consent (the Netherlands) or commercially acquired (AllCells, China or Milestone Shanghai Biological Science & Technology, China). Bone marrow mononuclear cells (BMMC) from patients with MM or patients with AML were isolated from bone marrow samples by density gradient centrifugation, obtained after approval by the institutional review board (medical ethical committee Amsterdam UMC, VUmc) and written informed consent from the patients or commercially acquired (MM samples from Cureline tissue bank, San Francisco, USA; AML samples from Centre de Resources Biologiques des Hospices Civils, Lyon, France). PBMC from patients with CLL were isolated from venous blood by density gradient centrifugation, obtained after approval by the institutional review board (medical ethical committee Amsterdam UMC, AMC) and written informed consent from the patients.

### Flow cytometry

All antibodies were used at empirically determined dilution factors. Staining was performed at 4°C in PBS supplemented with 0.1% BSA and 0.02% sodium azide unless otherwise stated. Analyses and cell sorting were performed using a FACS Canto II or FACS LSRFortessa (BD Biosciences). Data analysis was performed using Kaluza (Beckman Coulter), FCAP array and FlowJo (BD Biosciences).

### Generation of bs-constructs and binding assay

Human CD1d-specific and human Vδ2-specific VHH were generated and screened as previously described (Lameris et al. (2016) Immunology 149:111; de Bruin et al. (2016) Clin. Immunol 169:128). To generate CD1d-Vδ2 TCR bispecific VHHs, CDld (clone 1D12) and Vδ2 TCR (clone 5C8 (WO2015156673)) VHHs were linked by a Gly₄Ser(G₄S)-linker (VHH1D12-VHH5C8 (further referred to as CD1d-Vδ2 bsTCE)) and purified, tagless, and endotoxin free CD1d-Vδ2 bsTCE was produced by UPE, the Netherlands.

For *in vitro* binding experiments of monovalent VHH or bsTCE, ~1×10⁵ cells (Vγ9Vδ2-T, αβ-T, MM.1s WT or MM.1s.CD1d cells were incubated with PBS (negative control) or 1000 nM CD1d-Vδ2 bsTCE for 30-45 min at 4°C, followed by extensive washing and incubation for 30 min at 4 °C with GaL-FITC and analyzed by flow cytometry.

### Primary type 1 NKT and Vγ9Vδ2-T cell lines

Primary human type 1 NKT cells were generated and expanded as described previously (Lameris et al. (2014) Methods Mol Biol 1149:155). Primary human Vγ9Vδ2-T cells were generated and expanded as described previously with the modification that cells were cultured with RPMI-1640 supplemented with 10% (v/v) fetal calf serum, 0.05 mM β-mercaptoethanol, 100 IU ml⁻¹ sodium penicillin, 100 µg ml⁻¹ streptomycin sulphate, 2.0 mM L-glutamine, 10 IU ml⁻¹ rhIL-7, 10 ng ml⁻¹ rhIL-15 and 50 ng ml⁻¹ phytohaemagglutinin (PHA). Expanded type 1 NKT and Vγ9Vδ2-T cells selected for experiments were > 90% TRAV10⁺TRBV25-1⁺ and TRGV9⁺TRDV2⁺, respectively.

### Type 1 NKT and Vγ9Vδ2-T cell stimulation assay

To evaluate induction of degranulation of type 1 NKT and Vγ9Vδ2-T cells, target cells (MM.1s.CD1d, A-431) were co-cultured with medium (negative control), VHH1D12 or bsTCE at the indicated concentration, and with 5×10⁴type 1 NKT cells or 5×10⁴ Vγ9Vδ2-T cells for 4 h in the presence of PE-labelled anti-CD107a, after which type 1 NKT (CD3⁺CD45⁺TRGV9⁻TRAV-10⁺( CD1d(α-GalCer)-tetramer⁺)) and Vγ9Vδ2-T cells (CD3⁺CD45⁺TRGV9⁺TRAV-10⁻((TRDV2⁺)(CD1d(α-GalCer)-tetramer ^{/+})) were analyzed for CD107a expression. Supernatants from 24 h co-cultures of 1×10⁵ MM.1s.CD1d and 5×10⁴ type 1 NKT or Vγ9Vδ2-T cells with medium (negative control) or CD1d-Vδ2 bsTCE (50 nM) were analyzed for cytokine production by cytometric bead array (CBA).

### Cytotoxicity, and effector cell proliferation assay

The ability of human type 1 NKT and Vγ9Vδ2-T cells to lyse target cells was determined by flow cytometry. 1×10⁵ target cells (CCRF-CEM (whether or not stained with PBSE) were incubated with 5×10⁴ type 1 NKT or Vγ9Vδ2-T cells with medium (negative control) or bsTCE at indicated concentration. After 16 h incubation, the cells were stained with an antibody cocktail washed in annexin V buffer and briefly incubated with annexin V FITC, 7-AAD and flow cytometric counting beads, for subsequent flow cytometry analysis. Absolute number of living target cells (PBSE^{-/+}CD3^{-/dim}(CD7⁺)TRAV10⁻TRGV9⁻annexin V⁻ 7-AAD⁻) was determined according to the manufacturer's instructions. Specific lysis of target cells was calculated as follows; 100 - ((absolute cell number [condition] / absolute cell number [target cells only]) × 100).

### Patient-derived MM, AML and CLL samples

Using multicolor flow cytometry, CDld geometric median fluorescence (MF) was determined on MM (CD3⁻CD19^{-/+}CD38⁺CD45^{-/dim}CD56^{-/+}(CD81^{-/+})(CD117^{-/+})CD138⁺), AML (CD3⁻CD33^{-/+}CD34^{-/+}CD45^{dim/+}CD56^{-/+}CD117^{-/+}HLA-DR^{dim/+}) and CLL cells (CD3⁻CD5⁺CD19⁺). Geometric median fluorescence index (MFI) was calculated by dividing MF [stained tumor cells] by MF [unstained tumor cells or CDld negative population]. In addition, T cell percentage (CD3⁺CD45⁺) of mononuclear cells, type 1 NKT percentage (CD3⁺CD45⁺TRAV10⁺CD1d(α-GalCer)-tetramer⁺/TRBV25-1⁺) of CD3⁺ cells and Vγ9Vδ2-T percentage (CD3⁺CD45⁺TRGV9⁺TRDV2⁺) of CD3⁺ cells were determined using multicolor flow cytometry. For autologous and allogeneic type 1 NKT and Vγ9Vδ2-T cell degranulation, and cytotoxicity toward patient MM, monocytic AML and CLL cells, 1×10⁵ thawed BMMC (MM and AML) or PBMC (CLL, ± OCH (4 h, 100 ng ml⁻¹) pre-incubation) were cultured with medium (negative control) or CD1d-Vδ2 hu-bsTCE (50 nM) in the presence or absence of 5×10⁴ type 1 NKT or Vγ9Vδ2-T cells for 16 h in the presence of PE-labelled anti-CD107a, after which type 1 NKT (7-AAD⁻CD3⁺ (CD19⁻)(CD33⁻)CD45⁺(CD138⁻)TRGV9⁻TRAV-10⁺CD1d(α-GalCer)-tetramer⁺) and Vγ9Vδ2-T cells (7-AAD⁻CD3⁺(CD19⁻)(CD33⁻)CD45⁺(CD138⁻)TRGV9⁺TRAV-10⁻ ((TRDV2⁺)(CD1d(α-GalCer)-tetramer^{-/+})) were analyzed for CD107a expression. Live MM (7-AAD⁻CD3⁻(CD19⁻)CD45^{dim/-}CD38⁺(CD56^{-/+})CD138⁺), monocytic AML (7-AAD⁻CD3⁻CD33^{dim/+}(CD34⁺)CD45^{dim/+}(CD56⁺CD117⁺)HLA-DR^{dim/+}) and CLL cells (7-AAD⁻CD3⁻CD5⁺CD19⁺CD45⁺) were quantified by flow cytometry counting beads. Specific lysis was calculated as described above. The supernatants were collected and analyzed for cytokine production by CBA.

### Example 2: Type 1 NKT-cell and Vγ9Vδ2-T-cell activation by CD1d-Vδ2 bsTCE

A panel of Vγ9Vδ2-TCR-specific and CD1d-specific VHHs was generated via immunization of *Llama glama,* phage library construction, and clone selection (Lameris et al. (2016) Immunology 149:111; de Bruin et al. (2016) Clin. Immunol 169:128). We generated a bispecific antibody with dual-T cell engaging activity by fusing the CD1d-specific VHH antibody 1D12 with the Vδ2-TCR-specific VHH antibody 5C8. This molecule, termed CD1d-Vδ2 bsTCE, was designed to conditionally activate Vγ9Vδ2-T cells upon bispecific crosslinking, in addition to uniquely activating type 1 NKT cell by stabilizing TCR-CD1d interactions through 1D12 (Lameris et al. (2020) Nat. Cancer 1:1054). Binding experiments showed specific binding **(****figure 1A****)** and equivalent to that of the individual monospecific VHHs (not shown). We then evaluated the ability of CD1d-Vδ2 bsTCE to functionally engage type 1 NKT and Vγ9Vδ2-T cells by analyzing the expression of the degranulation maker CD107a. The bsTCE triggered robust degranulation of both type 1 NKT and Vγ9Vδ2-T cells in co-culture with CDld transfected MM cell line MM.1s (MM.1s.CD1d) with low-nM and low-pM EC₅₀ₛ, respectively **(****figure 1B****).** Importantly, in co-cultures with CD1d⁻ tumor cells (i.e. epidermoid carcinoma cell line A-431) no T cell degranulation was observed **(****figure 1C****).**

These data demonstrate that CD1d-Vδ2 bsTCE selectively binds to both CDld and the Vγ9Vδ2-TCR and induces target-dependent activation of both type 1 NKT and Vγ9Vδ2-T cells.

### Example 3: CD1d-Vδ2 bsTCE triggers cytokine release and anti-tumor activity of both type 1 NKT and Vγ9Vδ2-T cells

We next determined the ability of the CD1d-Vδ2 bsTCE to trigger cytokine release and cytotoxicity in co-cultures with CD1d-expressing tumor cells. The CD1d-Vδ2 bsTCE triggered TNF and IFN-γ secretion by both type 1 NKT and Vγ9Vδ2-T cells **(****figure 2A****).**

CD1d-Vδ2 bsTCE triggered strong type 1 NKT and Vγ9Vδ2-T cell-mediated anti-tumor activity as near-complete lysis of T-ALL cells was observed, with an EC₅₀ in the sub-nM range for type 1 NKT cells and in the low-pM range for Vγ9Vδ2-T cells **(****figure 2B****).**

### Example 4: CD1d-Vδ2 bsTCE triggers anti-tumor activity against patient-derived MM, monocytic AML and CLL cells

A humanized variant of CD1d-Vδ2 bsTCE, termed CD1d-Vδ2 hu-bsTCE, was prepared (SEQ ID NO: 9). Binding EC₅₀ for both CDld and Vγ9Vδ2-T cells and *in vitro* functional activity (degranulation EC₅₀ and tumor cell lysis) was identical between humanized and parental CD1d-Vδ2 bsTCE (not shown).

To evaluate the antitumor effects of CD1d-Vδ2 hu-bsTCE using patient-derived tumor samples, we focused on tumors of myelomonocytic and B cell lineage origin as these generally express CD1d. In bone marrow mononuclear cells (BMMC) of patients with MM and AML and PBMC of patients with CLL total T, type 1 NKT and Vγ9Vδ2-T cell frequencies and tumor cell CDld expression were assessed. In agreement with a previous report (Spanoudakis (2009) Blood 113, 2498), MM CDld expression was especially high in treatment naïve patients and slightly, though not statistically significantly, lower on tumor cells of patients who had received treatment **(****figure 3A****).** AML CDld expression was most pronounced on AML with a monocytic or myelomonocytic phenotype as compared to the remaining phenotypic AML subtypes **(****figure 3A****).** CDld was expressed by CLL cells in the majority of patients with no significant difference in treatment naïve versus treated patients **(****figure 3A****).** Upon 16 h culture of patient-derived MM, monocytic AML and CLL tumor samples with CD1d-Vδ2 hu-bsTCE, clear degranulation of patient Vγ9Vδ2-T cells was observed **(****figure 3B****).** Due to the (very) low type 1 NKT cell frequency in the tumor samples, degranulation of autologous type 1 NKT cells could not be reliably determined.

We then set out to evaluate the effect of CD1d-Vδ2 hu-bsTCE on patient-derived tumor samples using expanded healthy donor derived type 1 NKT and Vγ9Vδ2-T cells. In co-cultures of BMMC or PBMC from patients with MM, monocytic AML or CLL and Vγ9Vδ2-T cells, CD1d-Vδ2 hu-bsTCE induced robust Vγ9Vδ2-T cell degranulation **(****figure 3C-E).** Supernatants of tumor cells that were co-cultured with Vγ9Vδ2-T cells and CD1d-Vδ2 hu-bsTCE were enriched for Th1 type cytokines (IFN-γ) **(****figure 3C-E).** Importantly, in addition to degranulation and cytokine secretion, the CD1d-Vδ2 hu-bsTCE induced activation of Vγ9Vδ2-T cells also resulted in significant lysis of patient-derived MM, AML, and CLL tumor cells **(****figure 3C-E)**.

Of interest, while CD1d-Vδ2 hu-bsTCE efficiently triggered type 1 NKT cell degranulation, cytokine secretion and cytolytic activity when co-cultured with CD1d⁺ MM and AML cells **(****figure 3C** **and** **3D****),** in co-cultures with CD1d⁺ CLL the CD1d-Vδ2 hu-bsTCE only triggered minimal type 1 NKT-cell activation and cytolytic activity **(****figure 3E****).** However, pre-incubation of CLL cells with the low-affinity α-GalCer analog OCH clearly potentiated the ability of CD1d-Vδ2 hu-bsTCE to induce type 1 NKT cell degranulation, mixed Th1 and Th2 cytokine production, and cytotoxicity towards patient-derived CLL cells **(****figure 3F****)**. This indicates that the activity of CD1d-Vδ2 hu-bsTCE against CLL cells can be potentiated by an agonistic CDld ligand.

## Claims

1. A multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4 for use in the treatment of chronic lymphocytic leukemia in a human subject,
wherein said treatment further comprises administration of an agonistic CDld ligand.

2. The multispecific antibody for use according to claim 1, wherein a complex of human CDld and said agonistic CDld ligand presented on CD1d-expressing cells is capable of activating type 1 NKT cells.

3. The multispecific antibody for use according to any one of the preceding claims, wherein said agonistic CDld ligand is a glycolipid.

4. multispecific antibody for use according to claim 3, wherein said agonistic CDld ligand comprises the structure: 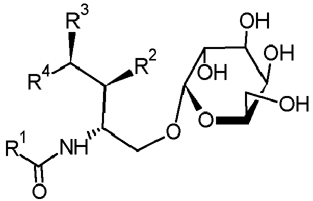
wherein R¹ is a C20-C30 alkyl group, preferably a linear C20-C30 alkyl group, e.g. a linear C25 group or a 11-(4-fluorophenyl)undecanoyl group,
wherein R² is H or OH,
wherein R³ is H or OH, and
wherein R⁴ is a C4-C18 alkyl group, preferably a linear C4-C18 alkyl group, e.g. a linear C5 alkyl group, a linear C14 alkyl group or a linear C15 alkyl group.

5. The multispecific antibody for use according to any one of the preceding claims, wherein the K_{D} for binding of a complex of human CDld loaded with said agonistic CDld ligand to a type 1 NKT T Cell Receptor is higher than the K_{D} for binding of a complex of human CDld loaded with alpha-GalCer to a type 1 NKT T Cell Receptor.

6. The multispecific antibody for use according to any one of the preceding claims, wherein said agonistic CDld ligand induces a more Th1-biased response by type 1 NKT cells compared to OCH.

7. The multispecific antibody for use according to any one of the preceding claims, wherein said agonistic CDld ligand induces a more Th2-biased response by type 1 NKT cells compared to alpha-C-GalCer.

8. The multispecific antibody for use according to claim 1 or 2, wherein said agonistic CDld ligand is selected from the group consisting of: alpha-GalCer, ThrCer, 7DW8-5 and OCH.

9. The multispecific antibody for use according to any one of the preceding claims, wherein said agonistic CDld ligand is capable of becoming loaded on CDld when CDld is located on the cell surface.

10. The multispecific antibody for use according to any one of the preceding claims, wherein said multispecific antibody binds the same epitope on CDld as the antibody set forth in SEQ ID NO:4.

11. The multispecific antibody for use according to any one of the preceding claims, wherein said second antigen-binding region comprises a single domain antibody.

12. The multispecific antibody for use according to any one of the preceding claims, wherein said multispecific antibody comprises the CDR1 sequence set forth in SEQ ID NO:1, the CDR2 sequence set forth in SEQ ID NO:2 and the CDR3 sequence set forth in SEQ ID NO:3, wherein, preferably, said multispecific antibody comprises a sequence that is at least 80%, such as at least 85%, e.g. at least 90%, such as at least 95%, e.g. at least 96%, such as at least 97%, e.g. at least 98%, such as at least 99%, or 100% identical to the sequence set forth in SEQ ID NO:4.

13. The multispecific antibody for use according to any one of the preceding claims, wherein said multispecific antibody binds the δ2 chain of the human Vγ9Vδ2-T cell receptor.

14. The multispecific antibody for use according to any one of the preceding claims, wherein said first antigen-binding region comprises a single domain antibody, wherein, preferably, said multispecific antibody comprises the CDR1 sequence set forth in SEQ ID NO:5, the CDR2 sequence set forth in SEQ ID NO:6 and the CDR3 sequence set forth in SEQ ID NO:7, wherein, more preferably, said multispecific antibody comprises a sequence that is at least 80%, such as at least 85%, e.g. at least 90%, such as at least 95%, e.g. at least 96%, such as at least 97%, e.g. at least 98%, such as at least 99%, e.g. 100% identical to the sequence set forth in SEQ ID NO:8, wherein, even more preferably, said multispecific antibody comprises or consists of the sequence set forth in SEQ ID NO:9.

15. The multispecific antibody for use according to any one of the preceding claims, wherein the treatment with said agonistic CDld ligand is initiated prior to, or simultaneously with, the treatment with the multispecific antibody.

16. A method for the treatment of chronic lymphocytic leukemia, comprising administration to a human subject in need thereof of:
(i) a multispecific antibody comprising a first antigen-binding region that binds a human Vγ9Vδ2-T cell receptor and a second antigen-binding region that binds human CDld and is able to compete for binding to human CDld with the antibody set forth in SEQ ID NO:4, and
(ii) an agonistic CDld ligand,
preferably, wherein the method comprises one or more of the further features set forth in claims 2 to 15.
